# EUROPÄISCHE PATENTSCHRIFT

(11) **EP 3 711 792 B1**
(45) Veröffentlichungstag und Bekanntmachung des Hinweises auf die Patenterteilung: **03.09.2025**
(21) Anmeldenummer: 20164528.0
(22) Anmeldetag: 20.03.2020
(51) Int. Cl.: A61M 5/14, A61M 5/168

(54) **TROPFKAMMERANORDNUNG FÜR EIN MEDIZINISCHES INFUSIONSGERÄT**
DRIP CHAMBER ASSEMBLY FOR A MEDICAL INFUSION DEVICE
AGENCEMENT DE CHAMBRE DE GOUTTE-À-GOUTTE POUR UN APPAREIL DE PERFUSION MÉDICALE

(30) Priorität: 21.03.2019 DE 102019203846
(43) Veröffentlichungstag der Anmeldung: 23.09.2020
(73) Patentinhaber: B. Braun Melsungen AG, 34212 Melsungen (DE)
(72) Erfinder: Conrad, Christoph, 34582 Borken (DE); Seidel, Gerrit, 34121 Kassel (DE); Fuchs, Jürgen, 34308 Bad Emstal (DE); Grundei, Ingo, 34225 Baunatal (DE)
(74) Vertreter: Patentanwälte Ruff, Wilhelm, Beier, Dauster & Partner mbB

(56) Entgegenhaltungen:
- DE-A1- 2 452 117
- FR-A2- 2 148 395
- GB-A- 1 554 629
- US-A- 4 361 147
- US-A1- 2011 125 103
- US-B1- 6 213 986

## Beschreibung

Die Erfindung betrifft eine Tropfkammeranordnung für ein medizinisches Infusionsgerät zur schwerkraftbetriebenen Überleitung einer Flüssigkeit bei einer Infusionstherapie, aufweisend ein Tropfkammergehäuse, das eine Tropfkammer zur Aufnahme der Flüssigkeit umgrenzt und einen Einlass zur Einleitung der Flüssigkeit in die Tropfkammer und einen Durchlass zur Ableitung der Flüssigkeit aus der Tropfkammer aufweist, und eine Stelleinrichtung, die eine manuelle Einstellung einer aus dem Durchlass der Tropfkammer abzuleitenden Flussrate der Flüssigkeit gestattet, wobei die Stelleinrichtung ein Betätigungselement, das relativ zu dem Tropfkammergehäuse zwischen unterschiedlichen Verlagerungspositionen beweglich an dem Tropfkammergehäuse gelagert ist, und einen einends fluidleitend mit dem Durchlass verbundenen und andernends in einen Austritt der Stelleinrichtung mündenden Fluidkanal mit einem Strömungswiderstand aufweist, und wobei der Strömungswiderstand mit dem Betätigungselement wirkverbunden und zur Einstellung der abzuleitenden Flussrate in Abhängigkeit der Verlagerungsposition des Betätigungselements veränderlich ist.

Eine derartige Tropfkammeranordnung ist aus der DE 20 2017 002 518 U1 bekannt und als Komponente eines medizinischen Infusionsgeräts zur Verwendung bei einer Infusionstherapie vorgesehen. Das medizinische Infusionsgerät kann auch als Infusionssystem oder Überleitungssystem bezeichnet werden und dient einer schwerkraftgetriebenen Überleitung einer medizinischen Flüssigkeit von einem die Flüssigkeit beinhaltenden Infusionsbehälter in einen patientenseitigen Zugang. Die bekannte Tropfkammeranordnung weist ein Tropfkammergehäuse auf, das eine Tropfkammer zur Aufnahme der Flüssigkeit umgrenzt und einen Einlass zur Einleitung und einen Durchlass zur Ableitung der Flüssigkeit aus der Tropfkammer aufweist. Zudem ist eine Stelleinrichtung unterseitig an dem Tropfkammergehäuse festgelegt und zur manuellen Einstellung einer aus dem Durchlass der Tropfkammer abzuleitenden Flussrate der Flüssigkeit vorgesehen. Die Stelleinrichtung weist ein Betätigungselement in Form eines Drehrades und einen Fluidkanal auf. Der Fluidkanal ist einends fluidleitend mit dem Durchlass der Tropfkammer verbunden und mündet andernends in einen Austritt der Stelleinrichtung. Der Fluidkanal weist einen Strömungswiderstand in Form einer kreisbogenförmigen Drosselnut auf. Zur Einstellung der abzuleitenden Flussrate ist eine strömungswirksame Länge der Drosselnut mittels einer Betätigung des Drehrads veränderlich. Eine weitere relevante Tropfkammeranordnung mit Stelleinrichtung ist aus der US 4 361 147 A bekannt.

Aufgabe der Erfindung ist es, eine Tropfkammeranordnung der eingangs genannten Art bereitzustellen, die gegenüber dem Stand der Technik eine vereinfachte Fertigung sowie Montage und gleichzeitig eine verbesserte Patientensicherheit ermöglicht.

Diese Aufgabe wird durch die erfinderische Tropfkammeranordnung gemäß Anspruch 1 gelöst. Dabei ist das Betätigungselement der Stelleinrichtung an dem Tropfkammergehäuse in Axialrichtung der Tropfkammer beweglich gelagert. Vorteilhaft ist auch, dass der Strömungswiderstand eine Blende ist, die einen in Abhängigkeit der Verlagerungsposition des Betätigungselements veränderlichen Querschnitt und eine unveränderliche Länge aufweist. Dadurch ist der veränderliche Strömungswiderstand lediglich lokal begrenzt in dem Fluidkanal ausgebildet. Zu diesem Zweck ist der Strömungswiderstand erfindungsgemäß als Blende ausgeführt, wobei der strömungswirksame Querschnitt der Blende mittels einer Betätigung des Betätigungselements einstellbar ist. Unter einer Blende soll eine lokale Verengung des Fluidkanals verstanden werden, deren strömungswirksame Länge im Vergleich zu wirkungsgleichen nicht lokal begrenzten Strömungswiderständen, wie beispielsweise längserstreckten Drosselkanälen, Kapillaren oder dergleichen, vergleichsweise kurz ist. Solche Drosselkanäle oder Kapillare weisen üblicherweise eine strömungswirksame Länge auf, die deutlich größer ist als deren strömungswirksamer Querschnitt. Aufgrund der sich hierdurch ergebenden vergleichsweise langen Kontaktlänge zwischen der Verengung und der Flüssigkeit kann ein Anhaften der Flüssigkeit an der Verengung und ein Verstopfen derselben unter gewissen Umständen nicht vermieden werden. Die erfindungsgemäße Lösung wirkt dem entgegen, da die Blende naturgemäß eine lediglich lokal begrenzte Verengung mit vergleichsweise kurzer strömungswirksamer Länge bildet. Durch die erfindungsgemäße Lösung kann somit insbesondere einem unerwünschten Verstopfen des Strömungswiderstands und damit des Fluidkanals entgegengewirkt werden. Hierdurch kann im Ergebnis eine verbesserte Patientensicherheit erreicht werden. Zudem ermöglicht die erfindungsgemäße Lösung gleichzeitig einen vereinfachten Aufbau der Tropfkammeranordnung und damit eine vereinfachte Fertigung, da insbesondere auf vergleichsweise aufwändig herzustellende Drosselkanäle und/oder Kapillare verzichtet werden kann. Sofern das Tropfkammergehäuse mehrteilig ausgebildet ist und beispielsweise ein einlassseitiges Tropfkammeroberteil und ein durchlassseitiges Tropfkammerunterteil aufweist, ist die Stelleinrichtung vorzugsweise an dem Tropfkammerunterteil angeordnet und/oder wenigstens abschnittsweise an demselben ausgebildet. Das Betätigungselement kann dreh-, schwenk- und/oder linearbeweglich an dem Tropfkammergehäuse gelagert sein. Demgemäß kann das Betätigungselement insbesondere in Form eines Dreh-, Schwenk- und/oder Schiebeelements ausgebildet sein. Der Durchlass der Tropfkammer mündet in den Fluidkanal der Stelleinrichtung, der seinerseits in den Austritt der Stelleinrichtung mündet. Vorzugsweise ist der Austritt zur Abgabe der Flüssigkeit in eine an der Stelleinrichtung festgelegte Schlauchleitung des Infusionsgeräts vorgesehen. Die Blende kann insbesondere eine Loch- oder Schlitzblende sein. Dementsprechend kann der veränderliche Querschnitt insbesondere loch- oder schlitzförmig gestaltet sein, wobei eine Loch- und/oder Schlitzkontur beliebig berandet und insbesondere eckig, rund oder oval sein kann. Die Veränderung des Querschnitts kann insbesondere mechanisch, beispielsweise mittels einer Relativbewegung den Querschnitt umgrenzender Abschnitte und/oder Bauteile der Stelleinrichtung bewirkt sein. Beispielsweise kann die Blende nach Art einer im Bereich der Optik allgemein bekannten Iris- oder Spaltsegmentblende ausgebildet sein. Alternativ kann die Veränderung des Querschnitts mittels einer Kontraktion und/oder Ausdehnung eines weichelastischen den Querschnitt umgrenzenden Abschnitts und/oder Bauteils der Stelleinrichtung bewirkt sein. Die Blende ist vorzugsweise mechanisch mit dem Betätigungselement wirkverbunden.

In Ausgestaltung der Erfindung beträgt die Länge der Blende maximal 10 %, bevorzugt maximal 5 %, und besonders bevorzugt maximal 1 %, einer Länge des Fluidkanals. Bei dieser Ausgestaltung der Erfindung ist die strömungswirksame, d.h. parallel zur Strömungsrichtung der Flüssigkeit erstreckte, Länge der Blende auf spezifische Weise lokal begrenzt und deutlich kürzer als die Länge des Fluidkanals. Bei einer Länge der Blende von maximal 10 % der Länge des Fluidkanals kann ein Verstopfen des Fluidkanals wirksam vermieden werden. Dies gilt umso mehr, wenn die Länge der Blende maximal 5 % der Länge des Fluidkanals beträgt. Sofern die Länge der Blende maximal 1 % der Länge des Fluidkanals beträgt, liegt ein lokal besonders eng begrenzter Strömungswiderstand vor.

Erfindungsgemäß ist die Blende mittels eines Zusammenwirkens einer zylindrischen Mantelfläche und eines zylindrischen Dichtabschnitts gebildet, wobei die Mantelfläche eine axial erstreckte Nut mit einem in Längsrichtung der Nut veränderlichen Nutquerschnitt aufweist, wobei der Dichtabschnitt koaxial zu der Mantelfläche orientiert ist und dieselbe radial kontaktierend in Umfangsrichtung umgreift, wobei ein an den Dichtabschnitt angrenzender strömungswirksamer Nutquerschnitt den Querschnitt der Blende bildet, und wobei die Mantelfläche und der Dichtabschnitt zur Veränderung des Querschnitts der Blende in Abhängigkeit der Verlagerungsposition des Betätigungselements zueinander axial relativbeweglich sind. Dies ermöglicht einen besonders einfachen Aufbau der hinsichtlich ihres Querschnitts veränderlichen Blende. Die zylindrische Mantelfläche mit der Nut kann dem Tropfkammergehäuse zugeordnet und dementsprechend feststehend sein und der Dichtabschnitt kann der Stelleinrichtung und/oder dem Betätigungselement zugeordnet und dementsprechend stellbeweglich sein oder umgekehrt. Die Nut ist in Axialrichtung der Mantelfläche erstreckt, was nicht zwingend bedeutet, dass die Nut parallel zur Axialrichtung der Mantelfläche erstreckt sein muss. Stattdessen kann die Nut auch schräg oder gekrümmt zur Axialrichtung der Mantelfläche verlaufen. Die Nut bildet eine Vertiefung der Mantelfläche und kann insbesondere einen runden, ovalen oder eckigen Querschnitt aufweisen. Dabei ist der Nutquerschnitt in Längsrichtung der Nut veränderlich. Sowohl die Mantelfläche als auch der Dichtabschnitt sind zueinander komplementär zylindrisch ausgebildet. Vorzugsweise sind die Mantelfläche und der Dichtabschnitt jeweils kreiszylindrisch ausgebildet. Der Dichtabschnitt umgreift die Mantelfläche in Umfangsrichtung, kontaktiert diese in radialer Richtung fluiddicht und übergreift hierbei auch einen Axialabschnitt der Nut. Ein hierbei zwischen dem Dichtabschnitt und der Mantelfläche im Bereich des Axialabschnitts gebildete Durchtritt bildet den Querschnitt der Blende. Werden nun die Mantelfläche und der Dichtabschnitt mittels einer Betätigung des Betätigungselements axial zueinander bewegt, ändert sich der jeweils strömungswirksame Axialabschnitt der Nut und damit - aufgrund des in Längsrichtung der Nut veränderlichen Nutquerschnitts - der Querschnitt der Blende. Sofern die Mantelfläche und der Dichtabschnitt jeweils kreiszylindrisch ausgebildet sind, kann die Relativbewegung zwischen der Mantelfläche und dem Dichtabschnitt neben der axialen Komponente auch eine rotatorische Komponente umfassen.

In weiterer Ausgestaltung der Erfindung ist der Dichtabschnitt an dem Tropfkammergehäuse ausgebildet, und die Mantelfläche ist an einem Stellzylinder ausgebildet, der dem Betätigungselement zugeordnet ist. Diese Ausgestaltung der Erfindung ermöglicht einen nochmals vereinfachten Aufbau und somit eine weiter vereinfachte Fertigung. Der Stellzylinder ist mit dem Betätigungselement wirkverbunden und vorzugsweise einstückig mit diesem ausgebildet. Dementsprechend ist der Stellzylinder mittels einer manuellen Betätigung des Betätigungselements relativ zu dem am Tropfkammergehäuse ausgebildeten und damit feststehenden Dichtabschnitt beweglich. Der Stellzylinder ist vorzugsweise ein Kreiszylinder.

In weiterer Ausgestaltung der Erfindung ist der Dichtabschnitt an einer Innenwandung eines röhrenförmigen Röhrenfortsatzes des Tropfkammergehäuses angeordnet, wobei der Stellzylinder koaxial in den Röhrenfortsatz eingeführt ist. Der Röhrenfortsatz ragt vorzugsweise koaxial von dem Tropfkammergehäuse ab. Sofern das Tropfkammergehäuse mehrteilig ausgebildet ist, ist der Röhrenfortsatz vorzugsweise an einem durchlassseitigen Tropfkammerunterteil ausgebildet. Der Dichtabschnitt ragt in Radialrichtung von der Innenwandung nach innen ab und kann vorzugsweise in Form eines ring- und/oder lippenförmigen Dichtelements ausgebildet sein.

In weiterer Ausgestaltung der Erfindung weist die Stelleinrichtung ein Stellgetriebe auf, mittels dessen eine rotatorische Bewegung des Betätigungselements in eine axiale Bewegung der Mantelfläche und/oder des Stellzylinders gegenüber dem Dichtabschnitt wandelbar ist. Demnach dient das Stellgetriebe einer Wandlung oder Umwandlung einer rotatorischen Betätigungsbewegung in eine axiale Stellbewegung. Dabei wirkt das Stellgetriebe vorzugsweise als Übersetzungsgetriebe, so dass eine dem Winkelbetrag nach große rotatorische Bewegung des Betätigungselements in eine dem Umfang nach vergleichsweise kleine oder große axiale Stellbewegung übersetzbar ist. Hierdurch kann die Flussrate besonders präzise eingestellt werden. Dies wirkt einer Unter- oder Überdosierung der Flüssigkeit entgegen, so dass eine nochmals verbesserte Patientensicherheit erreicht wird. Bei dieser Ausgestaltung der Erfindung ist das Betätigungselement rotatorisch beweglich an dem Tropfkammergehäuse gelagert und kann beispielsweise in Form eines Drehknebels, -griffs oder -rads ausgebildet sein.

In weiterer Ausgestaltung der Erfindung ist das Stellgetriebe ein Schraubgetriebe, das einen an dem Tropfkammergehäuse angeordneten Gewindeabschnitt und einen an dem Betätigungselement angeordneten Gegengewindeabschnitt aufweist. Der Gewindeabschnitt und der Gegengewindeabschnitt sind zueinander komplementär ausgestaltet. Der Gewindeabschnitt kann ein Innengewinde tragen und der Gegengewindeabschnitt ein hierzu komplementäres Außengewinde oder umgekehrt. Zur Einstellung der Flussrate kann das Betätigungselement über den Gegengewindeabschnitt auf den Gewindeabschnitt auf- oder in diesen eingeschraubt werden, wobei die rotatorische Bewegung des Betätigungselements hierbei in eine axiale Bewegung zur Veränderung des Querschnitts der Blende umgesetzt wird.

In weiterer Ausgestaltung der Erfindung weist die Stelleinrichtung eine zur Anzeige einer eingestellten Flussrate eingerichtete Anzeigeeinrichtung mit einer Skale und einem Zeigerelement auf. Die Skale kann an einem feststehenden Abschnitt des Tropfkammergehäuses angeordnet sein und das Zeigerelement kann an dem hierzu relativbeweglichen Betätigungselement angeordnet sein oder umgekehrt. Je nach Verlagerungsposition des Betätigungselements zeigt das Zeigerelement auf einen anderen Bereich der Skale, so dass die eingestellte Flussrate dementsprechend an der Anzeigeeinrichtung ablesbar ist. Die Skale kann durch eine Abfolge von Teilstrichen und/oder Ziffern ausgebildet sein. Diese Ausgestaltung der Erfindung wirkt einer etwaigen Fehldosierung der Flüssigkeit bei der Infusionstherapie entgegen und ermöglicht somit eine nochmals verbesserte Patientensicherheit.

Die Erfindung betrifft zudem ein medizinisches Infusionsgerät zur schwerkraftgetriebenen Überleitung einer Flüssigkeit bei einer Infusionstherapie, gekennzeichnet durch eine Tropfkammeranordnung nach der vorstehenden Beschreibung.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in teilweise ab- und aufgeschnittener schematischer Seitenansicht eine Ausführungsform eines erfindungsgemäßen medizinischen Infusionsgeräts, das eine Ausführungsform einer erfindungsgemäßen Tropfkammeranordnung mit einer Stelleinrichtung aufweist,
- Fig. 2: in vergrößerter schematischer Perspektivdarstellung einen Abschnitt der Stelleinrichtung,
- Fig. 3: in schematischer Längsschnittdarstellung den Abschnitt der Stelleinrichtung nach Fig. 2,
- Fig. 4, 5: die Tropfkammeranordnung nach Fig. 1 im Bereich der Stelleinrichtung jeweils in abgeschnittener und leicht perspektivischer Längsschnittdarstellung in einem ersten Zustand (Fig. 4) und in einem zweiten Zustand (Fig. 5) und
- Fig. 6, 7: jeweils in schematischer Querschnittsdarstellung die Tropfkammeranordnung nach Fig. 1 entlang einer Schnittlinie VI-VI gemäß Fig. 1 im ersten Zustand (Fig. 6) und im zweiten Zustand (Fig. 7).

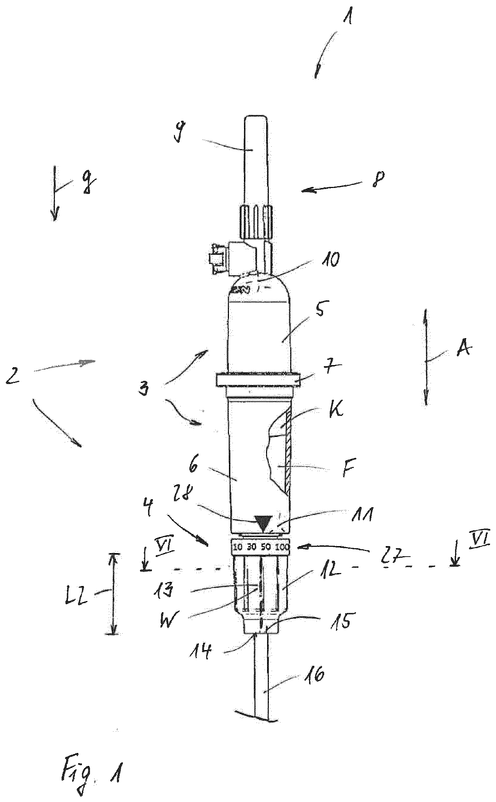

Gemäß Fig. 1 ist ein medizinisches Infusionsgerät 1 zur Verwendung bei einer Infusionstherapie vorgesehen. Das medizinische Infusionsgerät 1 kann auch als Infusionssystem oder Überleitungssystem bezeichnet werden und dient einer schwerkraftgetriebenen Überleitung einer nicht näher bezeichneten medizinischen Flüssigkeit F von einem die Flüssigkeit beinhaltenden Infusionsbehälter in einen patientenseitigen Zugang.

Das Infusionsgerät 1 weist eine Tropfkammeranordnung 2 mit einem Tropfkammergehäuse 3 und einer Stelleinrichtung 4 auf.

Das Tropfkammergehäuse 3 umgrenzt eine Tropfkammer K zur Aufnahme der Flüssigkeit F und ist vorliegend mehrteilig gestaltet, was jedoch nicht zwingend der Fall sein muss. Insoweit weist das Tropfkammergehäuse 3 vorliegend ein Tropfkammeroberteil 5 und ein Tropfkammerunterteil 6 auf. Das Tropfkammeroberteil 5 und das Tropfkammerunterteil 6 sind zur Ausbildung der Tropfkammer K stirnendseitig auf grundsätzlich bekannte Weise fluiddicht miteinander zusammengefügt. Zu diesem Zweck ist vorliegend eine Kunststoffumspritzung 7 vorgesehen, die zum einen eine fluiddichte Verbindung zwischen dem Tropfkammeroberteil 5 und dem Tropfkammerunterteil 6 gewährleistet und zum anderen einen nicht näher bezeichneten und in radialer Richtung der Tropfkammer K von dem Tropfkammergehäuse 3 im Übrigen abragenden Radialbund ausbildet.

Die Tropfkammeranordnung 2 weist vorliegend zudem ein Einstechteil 8 auf. Das Einstechteil 8 ist in dem anhand Fig. 1 ersichtlichen Zustand mit einer Abdeckkappe 9 versehen und dient einem Einstechen in einen hierfür vorgesehenen Abschnitt des besagten Infusionsbehälters. Das Einstechteil 8 weist eine grundsätzlich bekannte Gestaltung und Funktionsweise mit einem nicht näher ersichtlichen hohlen Einstechdorn auf, der zur Ableitung der Flüssigkeit aus dem Infusionsbehälter in diesen eingestochen wird. Hierdurch kann die Flüssigkeit aus dem Infusionsbehälter durch den Einstechdorn über einen Einlass 10 in die Tropfkammer K gelangen.

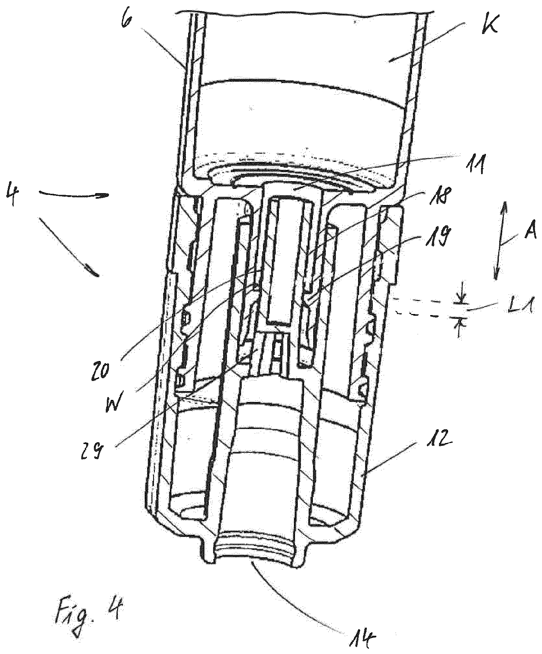

Die Stelleinrichtung 4 ist an einem dem Einstechteil 8 in Axialrichtung A der Tropfkammer K abgewandten Stirnendbereich des Tropfkammergehäuses 3 angeordnet. Die Stelleinrichtung 4 dient einer manuellen Einstellung einer aus einem Durchlass 11 der Tropfkammer K abzuleitenden Flussrate der Flüssigkeit F. Der insbesondere anhand Fig. 4 ersichtliche Durchlass 11 ist an einem - in Bezug auf die Zeichenebene der Fig. 1 - unteren Stirnendbereich des Tropfkammerunterteils 6 angeordnet und über die Tropfkammer K fluidleitend mit dem Einlass 10 verbunden. Die Stelleinrichtung 4 kann auch als Präzisionsflusssteller bezeichnet werden und gewährleistet, dass die Flüssigkeit F mit einer in medizinischer Hinsicht anforderungsgerechten Flussrate in den patientenseitigen Zugang verabreicht werden kann.

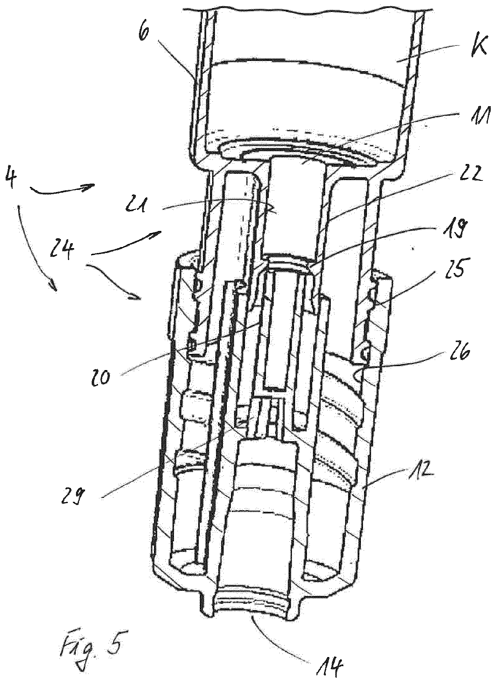

Die Stelleinrichtung 4 weist ein Betätigungselement 12 auf, das relativ zu dem Tropfkammergehäuse 3 zwischen unterschiedlichen Verlagerungspositionen (vgl. Fig. 4, 5) beweglich an dem Tropfkammergehäuse 3 gelagert ist. Das Betätigungselement 12 ist vorliegend auf noch näher beschriebene Weise dreh- bzw. schraubbeweglich an dem Tropfkammergehäuse 3 gelagert, was jedoch nicht zwingend ist. Stattdessen kann das Betätigungselement insbesondere in Form eines schwenkbeweglichen Schwenkelements oder eines linearverschieblichen Schiebeelements ausgebildet sein.

Die Stelleinrichtung 4 weist zudem einen Fluidkanal 13 auf, der anhand Fig. 1 schematisch stark vereinfacht strichpunktiert angedeutet ist und einends fluidleitend mit dem Durchlass 11 verbunden ist. Andernends mündet der Fluidkanal 13 in einen Austritt 14 der Stelleinrichtung 4. Der Austritt 14 ist seinerseits fluidleitend mit einem Einlass 15 einer Schlauchleitung 16 verbunden. Die Schlauchleitung 16 ist Bestandteil des Infusionsgeräts 1 und trägt an einem der Stelleinrichtung 4 abgewandten Stirnende einen nicht näher bezeichneten Patientenkonnektor zur fluidleitenden Verbindung mit dem besagten patientenseitigen Zugang.

Der Fluidkanal 13 weist einen mit dem Betätigungselement 12 wirkverbundenen Strömungswiderstand W auf, der zur Einstellung der abzuleitenden Flussrate in Abhängigkeit der Verlagerungsposition des Betätigungselements 12 veränderlich ist.

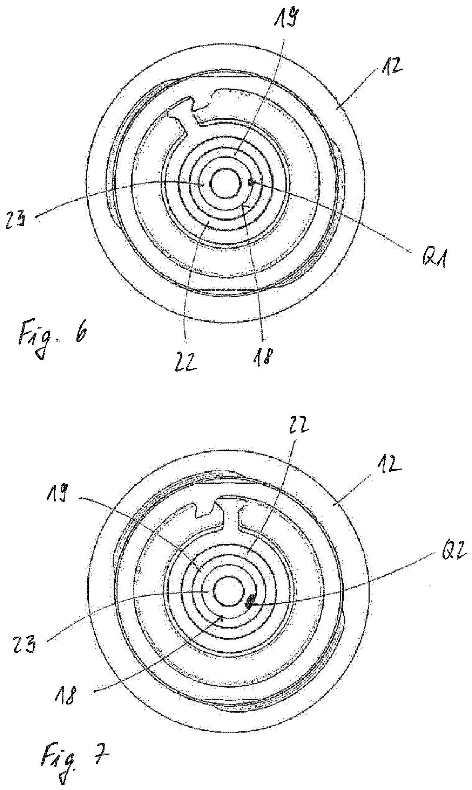

Der Strömungswiderstand ist eine Blende W, die einen in Abhängigkeit der Verlagerung des Betätigungselements 12 veränderlichen Querschnitt Q1, Q2 (vgl. Fig. 6, 7) und eine unveränderliche Länge L1 (Fig. 4) aufweist.

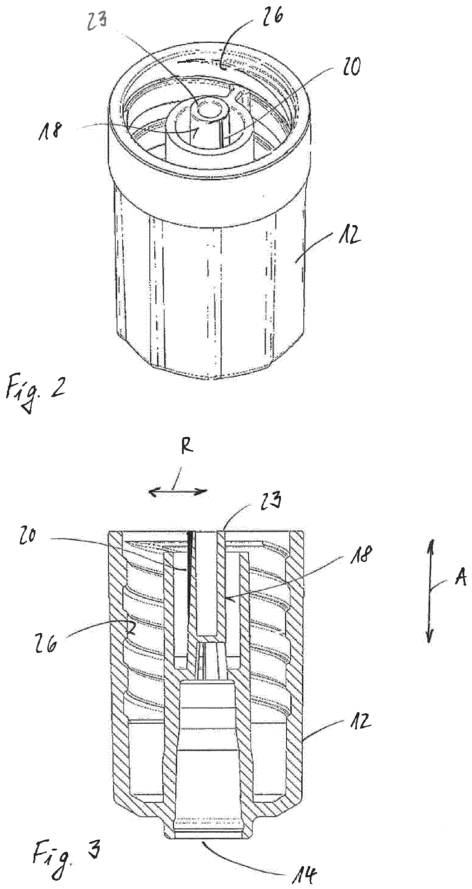

Die Blende W ist vorliegend mittels eines Zusammenwirkens einer zylindrischen Mantelfläche 18 (vgl. Fig. 2, 3) und eines zylindrischen Dichtabschnitts 19 (vgl. Fig. 4, 5) gebildet. Die Mantelfläche 18 weist eine Nut 20 auf, die in Axialrichtung A über die Mantelfläche 18 erstreckt ist. Vorliegend ist die Nut 20 parallel zur Axialrichtung A orientiert und insoweit in Form einer Längs- oder Axialnut ausgebildet, was jedoch nicht zwingend ist. Anhand Fig. 3 ist verdeutlicht, dass die Nut 20 einen in Längsrichtung der Nut 20 veränderlichen und nicht näher bezeichneten Nutquerschnitt aufweist. In der Längsschnittdarstellung der Fig. 3 ist die Nut 20 zur besseren Verdeutlichung schwarz eingefärbt. Hierdurch ist ersichtlich, dass der Nutquerschnitt ausgehend von einem - in Bezug auf die Zeichenebene der Fig. 3 - oberen Stirnendbereich der Nut 20 in Richtung eines unteren Stirnendbereichs kontinuierlich abnimmt. Mit anderen Worten ausgedrückt verringert sich eine in Radialrichtung R erstreckte Tiefe der Nut 20 in Axialrichtung A von oben nach unten, so dass die Nut 20 in Richtung des Durchlasses 11 (Fig. 4) einen maximalen Nutquerschnitt und in Richtung des Austritts 14 einen minimalen bzw. nicht vorhandenen Nutquerschnitt aufweist.

Der Dichtabschnitt 19 ist koaxial zu der Mantelfläche 18 orientiert (Fig. 4) und kontaktiert die Mantelfläche 18 in Radialrichtung R fluiddicht. Der Dichtabschnitt 19 bildet somit eine Art ringförmiges Dichtelement bzw. eine ringförmige Dichtlippe, die auf einem Außenumfang der Mantelfläche 18 anliegt. Dabei übergreift der Dichtabschnitt 19 auch die Nut 20, wobei ein in Radialrichtung R unterhalb des Dichtabschnitts 19 in Axialrichtung A über die Länge L1 erstreckte, nicht näher bezeichnete Axialabschnitt der Nut 20 eine fluidleitende Verbindung zwischen dem Durchlass 11 und dem Austritt 14 bildet. Der in diesem Bereich strömungswirksame Nutquerschnitt des Axialabschnitts bildet den Querschnitt Q1, Q2 der Blende W. Zur Veränderung des strömungswirksamen Querschnitts Q1, Q2 der Blende W und damit zur Einstellung der Flussrate sind die Mantelfläche 18 und der Dichtabschnitt 19 in Abhängigkeit der Verlagerungsposition des Betätigungselements 12 zueinander axial relativbeweglich, was anhand der Fig. 4 und 5 gezeigt ist.

Der Dichtabschnitt 19 ist vorliegend an dem Tropfkammergehäuse 3 und insoweit feststehend ausgebildet. Demgegenüber ist die Mantelfläche 18 mit der Nut 20 mit dem Betätigungselement 12 wirkverbunden und mittels einer Drehbewegung des Betätigungselements 12 in Axialrichtung A relativ zu dem Dichtabschnitt 19 beweglich.

Der Dichtabschnitt 19 ist vorliegend an einer Innenwandung 21 eines röhrenförmigen Röhrenfortsatzes 22 des Tropfkammergehäuses 3 angeordnet. Der Röhrenfortsatz 22 ist koaxial zu der Tropfkammer K erstreckt und unterseitig an dem Tropfkammerunterteil 6 ausgebildet. Der Dichtabschnitt 19 ragt in Radialrichtung R nach innen von der Innenwandung 21 ab und bildet insoweit eine Verengung eines nicht näher bezeichneten durch den röhrenförmigen Röhrenfortsatz 22 erstreckten Lumens.

Die Mantelfläche 18 mit der Nut 20 ist vorliegend an einem Stellzylinder 23 ausgebildet, der mechanisch mit dem Betätigungselement 12 wirkverbunden ist. Vorliegend ist das Betätigungselement 12 gemeinsam mit dem Stellzylinder 23 einstückig in Form eines Kunststoffspritzgussteils gefertigt, was jedoch nicht zwingend ist.

Der Stellzylinder 23 ist in Axialrichtung A in den Röhrenfortsatz 22 eingeführt, so dass der Dichtabschnitt 19 in Radialrichtung R auf dem Stellzylinder 23 bzw. dessen Mantelfläche 18 aufliegt.

Vorliegend weist die Stelleinrichtung 4 zur Wandlung einer rotatorischen Betätigungsbewegung des Betätigungselements 12 in die axiale Stellbewegung des Stellzylinders 23 ein Stellgetriebe in Form eines Schraubgetriebes 24 auf. Das Schraubgetriebe 24 weist einen am Tropfkammergehäuse 3 angeordneten Gewindeabschnitt 25 und einen am Betätigungselement 12 angeordneten komplementären Gegengewindeabschnitt 26 auf. Der Gewindeabschnitt 25 trägt vorliegend ein nicht näher bezeichnetes Außengewinde. Dementsprechend trägt der Gegengewindeabschnitt 26 ein hierzu komplementäres Innengewinde. Der Gewindeabschnitt 25 und der Gegengewindeabschnitt 26 sind koaxial zu der Tropfkammer K und somit auch koaxial zu dem Stellzylinder 23 und dem Dichtabschnitt 19 orientiert.

Zudem weist die Stelleinrichtung 4 vorliegend eine Anzeigeeinrichtung 27, 28 zur Anzeige einer mittels der Stelleinrichtung 4 eingestellten Flussrate der Flüssigkeit F auf. Die Anzeigeeinrichtung 27, 28 weist eine Skale 27, die aus einer Abfolge zueinander benachbart angeordneter Ziffern gebildet ist, und ein Zeigerelement 28 auf. Die Skale 27 ist vorliegend an einem nicht näher bezeichneten Außenumfang des Betätigungselements 12 angeordnet. Das Zeigerelement 28 ist vorliegend dreieckig ausgebildet und an einem an die Skale 27 angrenzenden Abschnitt des Tropfkammergehäuses 3 angeordnet. Je nach Verlagerungsposition des Betätigungselements 12 zeigt das Zeigerelement 28 auf einen unterschiedlichen Bereich der Skale 27, so dass hierdurch eine entsprechende Flussrate für eine Bedienperson ablesbar ist.

Nachfolgend wird die Funktionsweise der Stelleinrichtung 4 bei einer Überleitung der Flüssigkeit mittels des Infusionsgeräts 1 näher beschrieben. Hierbei wird von der anhand Fig. 1 ersichtlichen Konfiguration des Infusionsgeräts 1 ausgegangen, in der die Axialrichtung A parallel zu einem Erdschwerevektor g orientiert ist.

Zusätzlich wird abweichend von der Darstellung der Fig. 1 davon ausgegangen, dass das Einstechteil 8 auf die vorbeschriebene Weise in den Infusionsbehälter eingestochen ist, so dass die Flüssigkeit F schwerkraftbedingt aus dem Infusionsbehälter über das Einstechteil 8 durch den Einlass 10 in die Tropfkammer K gelangen kann.

In der anhand Fig. 1 ersichtlichen Konfiguration nimmt das Betätigungselement 12 eine anhand Fig. 4 näher dargestellte Verlagerungsposition ein. In dieser ist das Betätigungselement 12 in Axialrichtung A weitestmöglich auf das Tropfkammergehäuse 3 aufgeschraubt. Dementsprechend kontaktiert der Dichtabschnitt 19 den Stellzylinder 23 in einem unteren Bereich der Nut 20. In diesem Bereich ist der Nutquerschnitt der Nut 20 minimal bzw. eine Nuttiefe ist nicht vorhanden (vgl. Fig. 3), so dass der Durchlass 11 fluiddicht verschlossen ist.

Zur fluidleitenden Freigabe des Durchlasses 11 und zur Einstellung der aus der Tropfkammer K abzuleitenden Flussrate der Flüssigkeit F wird das Betätigungselement 12 um seine Längsachse drehbeweglich betätigt und hierbei durch ein Zusammenwirken des Gewindeabschnitts 25 und des Gegengewindeabschnitts 26 in Axialrichtung A von dem Tropfkammergehäuse 3 hinwegbewegt. Hierdurch bewegt sich der Stellzylinder 23 in Axialrichtung A - und gleichzeitig zudem um die Längsachse des Betätigungselements 12 - relativ zu dem Dichtabschnitt 19. Je nach Verlagerungsposition des Betätigungselements 12 bzw. Stellposition des Stellzylinders 23 wird hierdurch ein anderer Axialabschnitt der Nut 20 unter den Dichtabschnitt 19 verlagert. Aufgrund des in Längsrichtung der Nut 20 veränderlichen Nutquerschnitts ändert sich hierbei der strömungswirksame Querschnitt Q1, Q2 der Blende W und somit der Strömungswiderstand des Fluidkanals 13 (vgl. Fig. 6, 7). In der anhand Fig. 5 ersichtlichen Konfiguration ist das Betätigungselement 12 in eine nicht näher bezeichnete Endlage verlagert, in der der Stellzylinder 23 außer Eingriff mit dem Dichtabschnitt 19 gerät. Hierdurch ist ein nicht näher bezeichneter Ringspalt zwischen dem Dichtabschnitt 19 und einem stirnendseitigen Bereich des Stellzylinders 23 gebildet.

In einer zeichnerisch nicht dargestellten Zwischenstellung zwischen den anhand Fig. 4 und 5 ersichtlichen Konfigurationen kann die Flüssigkeit F dementsprechend über den Durchlass 11 in eine radial zwischen dem Röhrenfortsatz 22 und dem Stellzylinder 23 gebildeten Ringspalt und von diesem durch den strömungswirksamen Nutquerschnitt der Nut 20 im Bereich des Dichtabschnitts 19 in einen weiteren Durchlass 29 der Stelleinrichtung 4 und von dort in den Austritt 14 gelangen.

Anhand Fig. 4 ist ersichtlich, dass die Länge L1 der Blende W deutlich geringer ist als eine Länge L2 des Fluidkanals 13. Vorliegend beträgt die Länge L1 lediglich in etwa 1 % der Länge L2, so dass die Blende W stark lokal begrenzt in dem Fluidkanal 13 wirkt. Es versteht sich, dass auch ein hiervon abweichendes Verhältnis zwischen den Längen L1 und L2 vorgesehen sein kann.

## Patentansprüche

1. Tropfkammeranordnung (2) für ein medizinisches Infusionsgerät (1) zur schwerkraftbetriebenen Überleitung einer Flüssigkeit (F) bei einer Infusionstherapie, aufweisend
- ein Tropfkammergehäuse (3), das eine Tropfkammer (K) mit einer Axialrichtung (A) zur Aufnahme der Flüssigkeit (F) umgrenzt und einen Einlass (10) zur Einleitung der Flüssigkeit in die Tropfkammer (K) und einen Durchlass (11) zur Ableitung der Flüssigkeit (F) aus der Tropfkammer (K) aufweist,
- und eine Stelleinrichtung (4), die eine manuelle Einstellung einer aus dem Durchlass (11) der Tropfkammer (K) abzuleitenden Flussrate der Flüssigkeit (F) gestattet,
- wobei die Stelleinrichtung (4) ein Betätigungselement (12), das relativ zu dem Tropfkammergehäuse (3) zwischen unterschiedlichen Verlagerungspositionen beweglich ist, und einen einends fluidleitend mit dem Durchlass (11) verbundenen und andernends in einen Austritt (14) der Stelleinrichtung (4) mündenden Fluidkanal (13) mit einem Strömungswiderstand (W) aufweist,
- wobei der Strömungswiderstand (W) mit dem Betätigungselement (12) wirkverbunden und zur Einstellung der abzuleitenden Flussrate in Abhängigkeit der Verlagerungsposition des Betätigungselements (12) veränderlich ist,
- wobei der Strömungswiderstand eine Blende (W) ist, die einen in Abhängigkeit der Verlagerungsposition des Betätigungselements (12) veränderlichen Querschnitt (Q1, Q2) und eine unveränderliche Länge (L1) aufweist,
- wobei die Blende (W) mittels eines Zusammenwirkens einer zylindrischen Mantelfläche (18) und eines zylindrischen Dichtabschnitts (19) gebildet ist, wobei die Mantelfläche (18) eine axial erstreckte Nut (20) mit einem in Längsrichtung der Nut (20) veränderlichen Nutquerschnitt aufweist, wobei der Dichtabschnitt (19) koaxial zu der Mantelfläche (18) orientiert ist und dieselbe radial kontaktierend in Umfangsrichtung umgreift, wobei ein an den Dichtabschnitt (19) angrenzender strömungswirksamer Nutquerschnitt den Querschnitt (Q1, Q2) der Blende (W) bildet, und wobei die Mantelfläche (18) und der Dichtabschnitt (19) zur Veränderung des Querschnitts (Q1, Q2) der Blende (W) in Abhängigkeit der Verlagerungsposition des Betätigungselements (12) zueinander axial relativbeweglich sind,
- **dadurch gekennzeichnet, dass** das Betätigungselement in Axialrichtung (A) beweglich an dem Tropfkammergehäuse (3) gelagert ist.

2. Tropfkammeranordnung (2) nach Anspruch 1, **dadurch gekennzeichnet, dass** die Länge (L1) der Blende (W) maximal 10 %, bevorzugt maximal 5 %, besonders bevorzugt maximal 1 %, einer Länge (L2) des Fluidkanals (13) beträgt.

3. Tropfkammeranordnung (2) nach Anspruch 1 oder 2, **dadurch gekennzeichnet, dass** der Dichtabschnitt (19) an dem Tropfkammergehäuse (3) ausgebildet ist, und dass die Mantelfläche (18) an einem Stellzylinder (23) ausgebildet ist, der dem Betätigungselement (12) zugeordnet ist.

4. Tropfkammeranordnung (2) nach einem der vorhergehenden Ansprüche, **dadurch gekennzeichnet, dass** der Dichtabschnitt (19) an einer Innenwandung (21) eines röhrenförmigen Röhrenfortsatzes (22) des Tropfkammergehäuses (3) angeordnet ist, wobei der Stellzylinder (23) koaxial in den Röhrenfortsatz (22) eingeführt ist.

5. Tropfkammeranordnung (2) nach einem der vorhergehenden Ansprüche, **dadurch gekennzeichnet, dass** die Stelleinrichtung (4) ein Stellgetriebe (24) aufweist, mittels dessen eine rotatorische Bewegung des Betätigungselements (12) in eine axiale Bewegung der Mantelfläche (18) und/oder des Stellzylinders (23) gegenüber dem Dichtabschnitt (19) wandelbar ist.

6. Tropfkammeranordnung (2) nach Anspruch 5, **dadurch gekennzeichnet, dass** das Stellgetriebe ein Schraubgetriebe (24) ist, das einen an dem Tropfkammergehäuse (3) angeordneten Gewindeabschnitt (25) und einen an dem Betätigungselement (12) angeordneten Gegengewindeabschnitt (26) aufweist.

7. Tropfkammeranordnung (2) nach einem der vorhergehenden Ansprüche, **dadurch gekennzeichnet, dass** die Stelleinrichtung (4) eine zur Anzeige einer eingestellten Flussrate eingerichtete Anzeigeeinrichtung (27, 28) mit einer Skale (27) und einem Zeigerelement (28) aufweist.

8. Medizinisches Infusionsgerät (1) zur schwerkraftgetriebenen Überleitung einer Flüssigkeit (F) bei einer Infusionstherapie, **gekennzeichnet durch** eine Tropfkammeranordnung (2) nach einem der vorhergehenden Ansprüche.

## Claims

1. Drip chamber assembly (2) for a medical infusion device (1) for gravitational transfer of a liquid (F) in infusion therapy, having
- a drip chamber housing (3), which delimits a drip chamber (K) with an axial direction (A) for receiving the liquid (F) and which has an inlet (10) for introducing the liquid into the drip chamber (K) and a passage (11) for discharging the liquid (F) from the drip chamber (K),
- and an adjustment device (4) which permits manual adjustment of a flow rate of the liquid (F) that is to be discharged from the passage (11) of the drip chamber (K),
- wherein the adjustment device (4) has an actuation element (12), which is movable relative to the drip chamber housing (3) between different displacement positions, and a fluid channel (13) which at one end is connected in a fluid-conducting manner to the passage (11) and at the other end opens into an outlet (14) of the adjustment device (4), the fluid channel having a flow resistance (W),
- wherein the flow resistance (W) is operatively connected to the actuation element (12) and is variable, depending on the displacement position of the actuation element (12), for adjusting the flow rate that is to be discharged,
- wherein the flow resistance is a diaphragm (W) having a cross section (Q1, Q2), which is variable depending on the displacement position of the actuation element (12), and a non-variable length (L1),
- wherein the diaphragm (W) is formed by means of an interaction of a cylindrical lateral surface (18) and a cylindrical sealing portion (19), wherein the lateral surface (18) has an axially extending groove (20) with a groove cross section that is variable in the longitudinal direction of the groove (20), wherein the sealing portion (19) is oriented coaxially to the lateral surface (18) and engages circumferentially around same with radial contact, wherein a flow-effective groove cross section adjacent to the sealing portion (19) forms the cross section (Q1, Q2) of the diaphragm (W), and wherein the lateral surface (18) and the sealing portion (19) are axially movable relative to each other, depending on the displacement position of the actuation element (12), in order to change the cross section (Q1, Q2) of the diaphragm (W),
- **characterized in that** the actuation element is mounted on the drip chamber housing (3) movably in the axial direction (A).

2. Drip chamber assembly (2) according to Claim 1, **characterized in that** the length (L1) of the diaphragm (W) is at most 10%, preferably at most 5%, particularly preferably at most 1%, of a length (L2) of the fluid channel (13).

3. Drip chamber assembly (2) according to Claim 1 or 2, **characterized in that** the sealing portion (19) is formed on the drip chamber housing (3), and **in that** the lateral surface (18) is formed on an adjusting cylinder (23), which is assigned to the actuation element (12).

4. Drip chamber assembly (2) according to any one of the preceding claims, **characterized in that** the sealing portion (19) is arranged on an inner wall (21) of a tubular tube extension (22) of the drip chamber housing (3), wherein the adjusting cylinder (23) is inserted coaxially into the tube extension (22).

5. Drip chamber assembly (2) according to any one of the preceding claims, **characterized in that** the adjustment device (4) has an adjustment gearing (24), by means of which a rotational movement of the actuation element (12) is convertible into an axial movement of the lateral surface (18) and/or of the adjusting cylinder (23) with respect to the sealing portion (19).

6. Drip chamber assembly (2) according to Claim 5, **characterized in that** the adjustment gearing is a helical gear (24), which has a threaded portion (25) arranged on the drip chamber housing (3) and a mating threaded portion (26) arranged on the actuation element (12).

7. Drip chamber assembly (2) according to any one of the preceding claims, **characterized in that** the adjustment device (4) has a display device (27, 28), with a scale (27) and a pointer element (28), for displaying a set flow rate.

8. Medical infusion device (1) for gravitational transfer of a liquid (F) in infusion therapy, **characterized by** a drip chamber assembly (2) according to any one of the preceding claims.

## Revendications

1. Agencement de chambre compte-gouttes (2) pour un appareil de perfusion médical (1) pour le transfert par gravité d'un liquide (F) lors d'une thérapie par perfusion, présentant
- un boîtier de chambre compte-gouttes (3) qui délimite une chambre compte-gouttes (K) ayant une direction axiale (A) pour recevoir le liquide (F) et présente une entrée (10) pour introduire le liquide dans la chambre compte-gouttes (K) et un passage (11) pour évacuer le liquide (F) de la chambre compte-gouttes (K),
- et un dispositif de réglage (4) qui permet de régler manuellement un débit du liquide (F) à évacuer du passage (11) de la chambre compte-gouttes (K),
- le dispositif de réglage (4) présentant un élément d'actionnement (12) qui est mobile par rapport au boîtier de chambre compte-gouttes (3) entre différentes positions de déplacement, et un canal de fluide (13) ayant une résistance à l'écoulement (W), relié à une extrémité au passage (11) de manière à conduire le fluide et débouchant à l'autre extrémité dans une sortie (14) du dispositif de réglage (4),
- la résistance à l'écoulement (W) étant reliée fonctionnellement à l'élément d'actionnement (12) et étant variable pour ajuster le débit à évacuer en fonction de la position de déplacement de l'élément d'actionnement (12),
- la résistance à l'écoulement étant un diaphragme (W) qui présente une section transversale (Q1, Q2) variable en fonction de la position de déplacement de l'élément d'actionnement (12) et une longueur (L1) invariable,
- le diaphragme (W) étant formé au moyen d'une coopération d'une surface d'enveloppe cylindrique (18) et d'une section d'étanchéité cylindrique (19), la surface d'enveloppe (18) présentant une rainure (20) s'étendant axialement avec une section transversale de rainure variable dans la direction longitudinale de la rainure (20), la section d'étanchéité (19) étant orientée coaxialement à la surface d'enveloppe (18) et entourant celle-ci en contact radial dans la direction périphérique, une section transversale de rainure adjacente à la section d'étanchéité (19), agissant sur l'écoulement, formant la section transversale (Q1, Q2) du diaphragme (W), et la surface d'enveloppe (18) et la section d'étanchéité (19) pouvant être déplacées axialement l'une par rapport à l'autre pour modifier la section transversale (Q1, Q2) du diaphragme (W) en fonction de la position de déplacement de l'élément d'actionnement (12),
- **caractérisé en ce que** l'élément d'actionnement est monté de manière mobile dans la direction axiale (A) sur le boîtier de la chambre compte-gouttes (3).

2. Agencement de chambre compte-gouttes (2) selon la revendication 1, **caractérisé en ce que** la longueur (L1) du diaphragme (W) est d'au maximum 10 %, de préférence d'au maximum 5 %, de manière particulièrement préférée d'au maximum 1 %, d'une longueur (L2) du canal de fluide (13).

3. Agencement de chambre compte-gouttes (2) selon la revendication 1 ou 2, **caractérisé en ce que** la section d'étanchéité (19) est réalisée sur le boîtier de chambre compte-gouttes (3), et **en ce que** la surface d'enveloppe (18) est réalisée sur un cylindre de réglage (23) associé à l'élément d'actionnement (12).

4. Agencement de chambre compte-gouttes (2) selon l'une quelconque des revendications précédentes, **caractérisé en ce que** la section d'étanchéité (19) est agencée sur une paroi intérieure (21) d'un prolongement tubulaire en forme de tube (22) du boîtier de chambre compte-gouttes (3), le cylindre de réglage (23) étant inséré coaxialement dans le prolongement tubulaire (22).

5. Agencement de chambre compte-gouttes (2) selon l'une quelconque des revendications précédentes, **caractérisé en ce que** le dispositif de réglage (4) présente un mécanisme de réglage (24) au moyen duquel un mouvement de rotation de l'élément d'actionnement (12) peut être transformé en un mouvement axial de la surface d'enveloppe (18) et/ou du cylindre de réglage (23) par rapport à la section d'étanchéité (19).

6. Agencement de chambre compte-gouttes (2) selon la revendication 5, **caractérisé en ce que** le mécanisme de réglage est un mécanisme à vis (24) qui présente une section filetée (25) agencée sur le boîtier de chambre compte-gouttes (3) et une section filetée antagoniste (26) agencée sur l'élément d'actionnement (12).

7. Agencement de chambre compte-gouttes (2) selon l'une quelconque des revendications précédentes, **caractérisé en ce que** le dispositif de réglage (4) présente un dispositif d'affichage (27, 28) adapté pour indiquer un débit réglé, avec une échelle (27) et un élément indicateur (28).

8. Appareil de perfusion médical (1) pour le transfert par gravité d'un liquide (F) lors d'une thérapie par perfusion, **caractérisé par** un agencement de chambre compte-gouttes (2) selon l'une quelconque des revendications précédentes.
